# FASCICULE DE BREVET EUROPEEN

(11) **EP 1 465 560 B1**
(45) Date de publication et mention de la délivrance du brevet: **07.02.2007**
(21) Numéro de dépôt: 02806325.3
(22) Date de dépôt: 15.11.2002
(51) Int. Cl.: A61F 5/00, B22C 9/24, B29C 45/16

(54) **ANNEAU DE GASTROPLASTIE EN MATERIAU ELASTOMERE A DURETE VARIABLE**
AUS ELASTOMERMATERIAL VARIABLER HÄRTE HERGESTELLTER MAGENRING
GASTRIC RING MADE OF VARIABLE HARDNESS ELASTOMERIC MATERIAL

(30) Priorité: 15.01.2002 FR 0200517
(43) Date de publication de la demande: 13.10.2004
(73) Titulaire: Compagnie Europeenne d'Etude et de Recherche de Dispositifs pour l'Implantation par Laparoscopie, 38205 Vienne Cedex (FR)
(72) Inventeur: BENCHETRIT, Salomon, F-69300 Caluire et Cuire (FR)
(74) Mandataire: Martin, Didier Roland Valéry
(86) Numéro de dépôt international: PCT/FR2002/003933
(87) Numéro de publication internationale: WO 2003/059215

(56) Documents cités:
- EP-A- 0 254 366
- EP-A- 0 611 561
- EP-A- 0 769 282
- EP-A- 0 847 736
- WO-A-01/52777
- FR-A- 2 799 118

## Description

### DOMAINE TECHNIQUE

La présente invention se rapporte au domaine technique général des implants chirurgicaux destinés à traiter l'obésité par implantation d'une bande gastrique souple destinée à être fermée autour de l'estomac d'un patient, pour réduire le diamètre de l'ouverture du stoma en vue de contraindre le patient à réduire son alimentation.

La présente invention concerne un anneau de gastroplastie formé par une bande souple destinée à être fermée vers ses deux extrémités par des moyens de fermeture autour de l'estomac d'un patient, pour réduire le diamètre de l'ouverture du stoma, ladite bande comportant une chambre de compression annulaire à volume réglable reliée par un cathéter à un dispositif de réglage du diamètre de ladite chambre par injection ou retrait de fluide, ladite chambre étant délimitée par des parois comprenant un renfort dorsal prolongé par des parois latérales.

### TECHNIQUE ANTERIEURE

Dans le cas de patients atteints d'obésité extrêmement sévère (obésité morbide), c'est-à-dire dans le cas de patients dont le poids excède en général le poids idéal d'au moins 50 kilos par exemple, il est absolument nécessaire d'intervenir de manière chirurgicale afin d'éviter non seulement des problèmes graves de santé, mais encore pour éviter une mort quasi certaine et proche des patients.

En effet, il est acquis que les patients souffrant d'obésité morbide voient leur espérance de vie réduite de manière importante, et d'au moins une dizaine à une quinzaine d'années, tout en créant d'importants problèmes de charges psychologiques. Par ailleurs, on constate également l'apparition générale de problèmes de santé co-latéraux, tels que l'apparition de maladies cardiovasculaires ou l'apparition de phénomènes d'hypertensions, de diabète et d'arthrites sévères notamment.

Il est également connu que, dans le cas d'obésité extrêmement sévère, les traitements curatifs classiques basés sur une diète sévère combiné, par exemple, avec une série d'exercices physiques sont peu adaptés à ces cas d'obésités extrêmes.

C'est la raison pour laquelle les traitements efficaces et à long terme de l'obésité morbide font intervenir un traitement chirurgical.

De manière générale, on distingue des techniques de traitements chirurgicales faisant intervenir, un défaut d'absorption des aliments, c'est-à-dire un raccourcissement du passage classique de l'aliment et des sucs digestifs d'une part et les techniques faisant intervenir une restriction gastrique réduisant la taille de l'estomac d'autre part.

Les techniques chirurgicales impliquant un défaut d'absorption sont par exemple celles dans lesquelles on réalise un by-pass ou une dérivation du petit intestin ou encore celles mettant en oeuvre une séparation du passage des aliments relativement aux sucs digestifs. Ces techniques sont désormais rarement utilisées, car elles peuvent donner lieu à de sévères complications pour le patient et nécessitent dans tous les cas une importante intervention chirurgicale.

C'est la raison pour laquelle on tend désormais à privilégier les techniques chirurgicales mettant en oeuvre une restriction gastrique pour réduire la prise d'aliments.

Ces techniques largement connues font intervenir l'utilisation d'anneaux de gastroplastie implantés autour de l'estomac du patient, en vue de réduire sa taille ainsi que le diamètre de son passage (stoma).

La structure générale des anneaux de gastroplastie utilisés est bien connue et fait intervenir une bande souple, réalisée en matériau élastomère, destinée à être fermée vers deux extrémités par des moyens de fermeture autour de l'estomac d'un patient, pour réduire le diamètre de l'ouverture du stoma. Les moyens de fermeture sont généralement situés sur la partie externe ou dorsale de la bande souple, et font intervenir différents types de verrouillage, par exemple un verrouillage mécanique avec ou sans suturage. Les anneaux connus comportent également une bande avec une chambre de compression annulaire dont le volume ou l'expansion diamétrale est réglable, ladite chambre étant susceptible d'être reliée par un cathéter à un dispositif de réglage du diamètre de la chambre par injection ou retrait de fluide. Grâce à cette particularité, on peut ainsi, à partir d'un anneau de taille ou de diamètre fixe, régler finement le diamètre de l'anneau par injection ou retrait de fluide, ce qui provoque une expansion ou une rétraction diamétrale correspondante de l'anneau.

Les dispositifs connus du type mentionnés ci-dessus donnent généralement satisfaction mais souffrent d'un certain nombre de problèmes, et en particulier de problèmes de tolérance par le patient.

Il s'avère en effet particulièrement important de réduire autant que possible la sensation de gène procurée par de tels anneaux au niveau de la zone de restriction de l'estomac et d'éviter ou de réduire l'apparition de lésions cellulaires dans la zone de restriction.

Or, pour des raisons de conception, et en particulier de solidité, les anneaux de gastroplastie connus s'avèrent toujours source de gène et de lésions ou d'inflammations cellulaires au niveau de la zone de restriction. En effet, il s'avère nécessaire pour privilégier la solidité de tels anneaux, et en particulier pour assurer une bonne fiabilité de la fermeture de l'anneau, d'utiliser des matériaux élastomères de dureté Shore A élevée et donc de rigidité importante, ce qui certes contribue à renforcer la solidité de l'anneau, mais contribue également à le rendre source de traumatisme pour les tissus cellulaires et pour le patient.

En particulier, il s'avère que le positionnement sur la partie dorsale de l'anneau des moyens de fermeture, s'il permet d'obtenir un anneau dont la partie annulaire réglable entoure l'estomac sur 360 degrés, ce qui réduit le traumatisme des tissus, contribue néanmoins à faire exercer sur les moyens de fermeture externes des forces de tractions antagonistes particulièrement importantes, qu'il convient précisément de maîtriser en renforçant la rigidité générale de l'anneau. La conception des anneaux de l'art antérieur connu résulte donc d'un nombre important de compromis techniques difficiles à maîtriser, générant toujours un certain traumatisme pour le patient.

En outre, il s'avère que les procédés de fabrication mis en oeuvre pour réaliser de tels anneaux de gastroplastie sont difficiles à mettre en oeuvre, car ils font en général intervenir la fabrication d'un renfort dorsal de l'anneau avec les moyens de fermeture, renfort dorsal sur lequel est collée la chambre annulaire proprement dite de l'anneau. On conçoit que ce type de procédé puisse conduire à des risques non négligeables de décollage partiel des pièces collées et qu'un certain nombre de pièces défectueuses peuvent être détectées en cours de fabrication. Les dispositifs et procédés connus s'avèrent donc en générai difficiles à mettre en oeuvre et d'un coût industriel relativement élevé, si l'on souhaite obtenir des pièces présentant une bonne régularité et exemptes de tous défauts.

Le document FR-A-2 799 118 correspond au préambule de la revendication 1.

### EXPOSE DE L'INVENTION

L'objet assigné à l'invention vise en conséquence à proposer un nouvel anneau de gastroplastie permettant de porter remède aux différents inconvénients énumérés précédemment et qui soit particulièrement atraumatique et bien supporté par le patient, tout en étant d'une bonne robustesse, facile à fabriquer et d'un coût réduit.

Un autre objet de l'invention vise à proposer un nouvel anneau de gastroplastie particulièrement fiable dans sa tenue mécanique.

Un autre objet de l'invention vise à proposer un nouvel anneau de gastroplastie particulièrement robuste tout en étant particulièrement atraumatique.

Un autre objet de l'invention vise à proposer un nouvel anneau de gastroplastie dont les moyens de fermeture sont particulièrement résistants.

Un autre objet de l'invention vise à proposer un nouvel anneau de gastroplastie particulièrement simple à fabriquer.

L'objet assigné à l'invention vise également à proposer un nouveau procédé de fabrication d'un anneau de gastroplastie par injection d'un matériau élastomère dans un moule, ledit nouveau procédé étant particulièrement simplifié et rapide, tout en permettant d'obtenir un anneau de gastroplastie robuste et atraumatique.

Un autre objet de l'invention vise à proposer un nouveau procédé de fabrication particulièrement économique et permettant de réduire le nombre d'étapes de fabrication.

Un autre objet de l'invention vise à proposer un nouveau procédé de fabrication particulièrement adapté à la réalisation d'un anneau de gastroplastie monobloc.

Un autre objet de l'invention vise à proposer un nouveau procédé de fabrication permettant d'obtenir un anneau de gastroplastie particulièrement fiable et sûr.

Les objets assignés à l'invention sont atteints à l'aide d'un anneau de gastroplastie formé par une bande souple destinée à être fermée vers ses deux extrémités par des moyens de fermeture autour de l'estomac d'un patient, pour réduire le diamètre de l'ouverture du stoma, ladite bande comportant une chambre de compression annulaire à volume réglable reliée par un cathéter, à un dispositif de réglage du diamètre de ladite chambre par injection ou retrait de fluide, ladite chambre étant délimitée par des parois comprenant un renfort dorsal prolongé par des parois latérales, caractérisé en ce que :
- le renfort dorsal est réalisé à partir d'un premier matériau élastomère de dureté Shore A, prédéterminée d₁,
- les parois latérales sont réalisées à partir d'un second matériau élastomère de même nature que le premier matériau mais de dureté Shore A prédéterminée d₂, telle que d₂ < d₁. de manière à obtenir une chambre annulaire monobloc de dureté Shore A variable dans son épaisseur.

Les objets assignés à l'invention sont également atteints à l'aide d'un procédé de fabrication d'un anneau de gastroplastie par injection d'un matériau élastomère dans un moule pourvu d'au moins une empreinte avec au moins un noyau, caractérisé en ce que :
- a) on assure l'injection d'un premier matériau élastomère de dureté Shore A prédéterminée d₁ en vue de réaliser au moins le renfort dorsal de l'anneau ;
- b) puis on assure, par surmoulage sur au moins le renfort dorsal, l'injection d'un second matériau élastomère de même nature que le premier matériau mais de dureté Shore A prédéterminée d₂, avec d₂ < d₁, en vue de réaliser les parties restantes de l'anneau et obtenir un anneau monobloc surmoulé à dureté variable.

### DESCRIPTIF SOMMAIRE DES DESSINS

D'autres objets et avantages de l'invention apparaîtront mieux à la lecture de la description ci-jointe, ainsi qu'à l'aide des dessins annexés, à titre purement illustratif et informatif, dans lesquels :
- La figure 1 illustre, selon une vue latérale, un anneau de gastroplastie conforme à l'invention dans sa position desserrée.
- La figure 2 illustre, selon une vue latérale identique à celle de la figure 1, un anneau de gastroplastie conforme à l'invention dans sa position fermée.
- La figure 3 illustre, selon une vue en coupe transversale, un anneau de gastroplastie conforme à l'invention en position de fermeture.
- La figure 4 illustre, selon une vue schématique, une étape du procédé de fabrication conforme à l'invention, dans laquelle on réalise le renfort dorsal de l'anneau à l'aide d'une empreinte dans laquelle est disposé un noyau de renfort dorsal.
- La figure 5 illustre une étape du procédé de fabrication selon l'invention, dans laquelle on réalise une partie des moyens de fixation de l'anneau dans une empreinte du moyen de fermeture dans laquelle est disposé un noyau de bague.
- La figure 6 illustre une étape du procédé de fabrication conforme à l'invention, dans laquelle on assure le surmoulage sur le renfort dorsal et le noyau de bague de l'anneau du second matériau élastomère dans un moule d'anneau pour obtenir l'anneau définitif.
- La figure 7 illustre, selon une vue en perspective, le noyau de renfort dorsal supportant le renfort dorsal de l'anneau tel qu'obtenu à l'issue de l'étape d'injection illustrée à la figure 4.
- La figure 8 illustre, selon une vue en perspective, le noyau de bague supportant la bague de renfort, tel qu'obtenu à l'issue de l'étape d'injection illustrée à la figure 5.
- La figure 9 illustre, selon une vue en coupe transversale, la forme particulière de la section de la zone dorsale de l'anneau.

### MEILLEURE MANIERE DE REALISER L'INVENTION

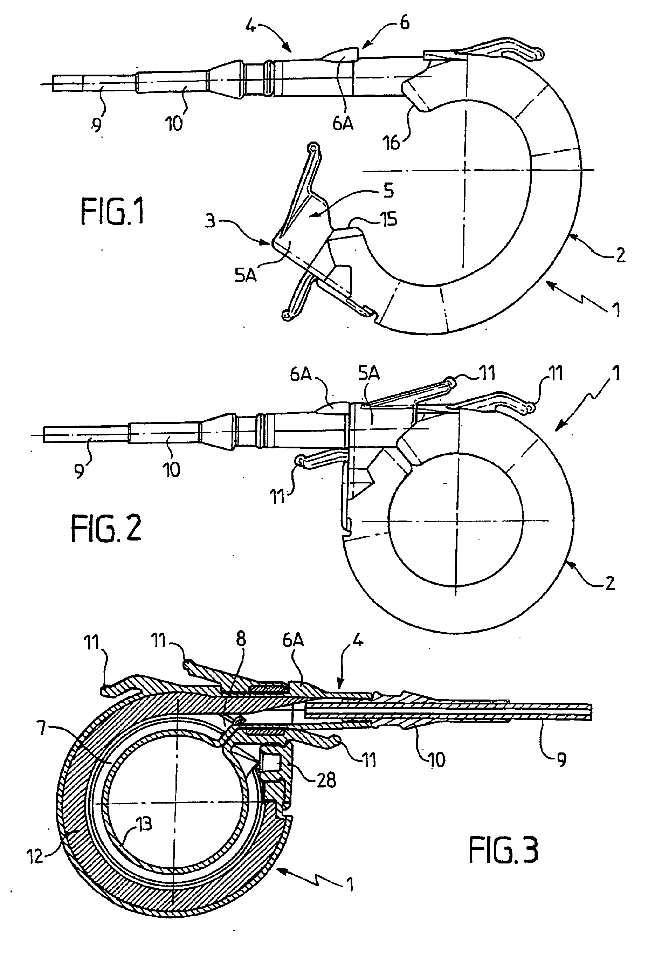

Les figures 1 à 3 illustrent un anneau de gastroplastie 1 conforme à l'invention formé par une bande souple 2 réalisée à partir d'un matériau élastomère, par exemple du silicone, destinée à être fermée sensiblement vers ses deux extrémités 3, 4 par des moyens de fermeture 5, 6 autour de l'estomac d'un patient, en vue de réduire le diamètre de l'ouverture du stoma.

La position de fermeture de l'anneau est illustrée à la figure 2, position dans laquelle les moyens de fermeture 5, 6 coopèrent entre eux pour assurer le verrouillage de l'anneau 1.

L'anneau 1 conforme à l'invention comporte également intérieurement une chambre de compression 7 s'étendant sur la majeure partie de la longueur de la bande souple 2, de telle façon qu'en position de fermeture elle forme une chambre de compression annulaire 7 susceptible d'enserrer l'estomac sur une plage angulaire égale ou sensiblement égale à 360 degrés.

De manière connue, la chambre annulaire 7 est à volume réglable, c'est-à-dire que son expansion diamétrale peut être réglée en expansion ou en rétraction, de manière à régler concomitamment le diamètre de l'ouverture du stoma. A cette fin, la chambre de compression annulaire 7 est reliée par l'ouverture 8 et par un cathéter 9 associé à l'ouverture 8, à un dispositif de réglage (non représenté aux figures) du diamètre de ladite chambre par injection ou retrait de fluide. De manière connue, le dispositif de réglage est formé par un boîtier miniaturisé qui peut être implanté sous la peau du patient, le boîtier comportant une membrane auto-obturante destinée à être percée par une seringue permettant d'injecter ou de retirer une certaine quantité de fluide (en général de l'eau physiologique) servant à assurer la variation de volume de la chambre de compression annulaire 7.

Tel qu'illustré aux figures 1 à 3, le cathéter 9 peut être relié à la bande souple 2 par l'intermédiaire d'un organe de connexion, tel qu'un embout 10. Accessoirement, l'anneau de gastroplastie 1 conforme à l'invention peut être pourvu d'une ou plusieurs languettes 11 de préhension disposées à des endroits prédéterminés, par exemple vers les extrémités 3, 4, de manière à faciliter la manipulation de l'anneau, et en particulier sa fermeture, et surtout son ouverture ou déverrouillage.

Tel que cela est également connu, l'anneau de gastroplastie 1 conforme à l'invention comporte une chambre 7 qui est délimitée par des parois comprenant un renfort dorsal 12 prolongé en direction du centre théorique de l'anneau lorsqu'il est fermé, et de chaque côté par des parois latérales 13, avantageusement d'épaisseur inférieure à l'épaisseur du renfort dorsal 12. On aboutit ainsi à un anneau de gastroplastie 1 dont la rigidité de la partie dorsale extérieure de l'anneau 1 est de rigidité supérieure à la partie interne de l'anneau qui est en contact avec les tissus de l'estomac.

Avantageusement, la section transversale du renfort dorsal sera sensiblement en forme de U, l'âme 12A du U formant la partie typiquement dorsale de l'anneau et étant d'une épaisseur supérieure à celle des branches 12B du U (figure 9).

Selon des caractéristiques importantes de l'invention, le renfort dorsal 12 est réalisé à partir d'un premier matériau élastomère de dureté Shore A, prédéterminée d1, alors que les parois latérales 13 sont elles réalisées à partir d'un second matériau élastomère de même nature que le premier matériau, mais de dureté Shore A prédéterminée d2, tel que d2 < d1, de manière à obtenir une chambre annulaire 7 monobloc de dureté Shore A variable dans l'épaisseur de ses parois.

Au sens de l'invention, on entendra par l'expression *«matériau élastomère de même nature»* un matériau de composition chimique très proche, voire similaire ou identique, ces matériaux ne différant entre eux de manière significative que par leur caractéristique de rigidité.

Grâce à l'utilisation de matériaux de duretés différentes, on obtient une meilleure maîtrise de la déformation de la chambre annulaire 7, en particulier dans sa partie interne en contact avec l'estomac, la compression de l'estomac étant de plus particulièrement douce et donc bien supportée. Par ailleurs, l'anneau 1 obtenu est particulièrement résistant, puisque la majeure partie des efforts mécaniques de traction est supportée par le renfort dorsal 12 qui supporte les moyens de fermetures 5, 6 et qui est de rigidité supérieure.

L'effet combiné d'un renfort dorsal et d'une chambre annulaire en élastomère de dureté différente permet, entre autre, d'obtenir une plage d'ajustage sur le diamètre interne de l'anneau importante avec l'avantage de simplifier le choix d'un anneau en ramenant celui-ci à une seule taille.

Le recours à l'utilisation de deux matériaux élastomères de même nature permet d'obtenir la chambre annulaire 7 par une opération de surmoulage des deux matériaux élastomères, les parois latérales 13 étant surmoulées sur le renfort dorsal 12. On obtient ainsi une excellente cohésion continue des matériaux élastomères de même nature.

Tel qu'illustré à la figure 9, les branches 12B. facilitent la liaison lors du surmoulage avec le matériau de dureté inférieure formant les parois latérales 13, tout en facilitant le positionnement des pièces lors du surmoulage. La forme spécifique en U du renfort dorsal 12, avec son âme 12A formant une surépaisseur en matériau à dureté augmentée, conjuguée aux parois latérales 13 minces de dureté inférieure conduit à obtenir une chambre de compression annulaire 7, dont la compression est majoritairement sinon exclusivement centripète (flèche F, Figure 9), *i.e.* dirigée vers le centre théorique de l'anneau. En effet, la chambre se déforme surtout à partir des parois latérales 13 de dureté et d'épaisseur faibles en comparaison avec l'âme 12A qui ne se déforme pas ou peu.

Grâce à cette technique, on obtient une excellente solidarisation des parois mêmes de la chambre annulaire 7, ce qui est un gage de sécurité, tout en facilitant sa fabrication. Il est également particulièrement facile de réaliser un anneau de gastroplastie 1 conforme à l'invention à l'aide de deux simples étapes de surmoulages, correspondant à une injection de deux matériaux élastomères de même nature, pour obtenir un anneau monobloc bi-matériaux surmoulé avec un renfort dorsal réalisé en un premier matériau élastomère de dureté Shore A d1 supérieure à la dureté Shore A du second matériau élastomère constituant le reste de l'anneau, à savoir les parois latérales de la chambre annulaire 7, les moyens de fermeture 5, 6 et avantageusement les languettes 11, le cathéter 9 et l'embout 10.

Avantageusement, la valeur de d1 sera comprise entre 65 et 85 Shore A, la valeur de d2 étant comprise entre 25 et 45 Shore A. De manière particulièrement avantageuse, la valeur de d1 sera de l'ordre de 80 Shore A, et la valeur d2 étant de l'ordre de 30 Shore A.

Selon une version particulièrement avantageuse de l'invention, les moyens de fermeture 5, 6 sont également réalisés en matériaux élastomères et disposés sur la partie dorsale de l'anneau, c'est-à-dire à l'extérieur de l'anneau lorsque ce dernier occupe sa position de fermeture, telle qu'illustrée à la figure 2.

De manière connue, les moyens de fermeture 5, 6 comprennent un moyen femelle 5A solidaire de l'extrémité 3 de la bande souple et formé par un manchon percé ou une bague. Les moyens de fermeture comprennent également un moyen mâle 6A solidaire de l'autre extrémité 4 de la bande souple 2, le moyen mâle 6A étant formé par exemple par une excroissance, sensiblement radiale, formant butée et par une zone susceptible de s'expanser sous l'effet de l'augmentation de la pression interne dans la chambre annulaire 7.

En position de fermeture, le cathéter 9 ainsi que l'embout 10 et l'extrémité 4 sont introduits à l'intérieur de la bague 5A, de telle manière que cette dernière coopère avec le moyen mâle 6A assurant la fermeture de l'anneau 1.

Selon une variante particulièrement avantageuse de l'invention, au moins une partie des moyens de fermeture 5, 6 est réalisée à partir du premier matériau élastomère. Selon une variante particulièrement avantageuse de l'invention, la bague 5A sera réalisée à partir du premier matériau élastomère présentant la dureté Shore A supérieure. Grâce à cette particularité, on obtient un renforcement non négligeable de la solidité de la fermeture de l'anneau, la partie 5A étant réalisée dans un matériau présentant la rigidité supérieure, alors que le moyen mâle 6, 6A peut être réalisé dans le matériau élastomère présentant la dureté inférieure, compte tenu de la nécessité d'expansion radiale qu'il doit effectuer.

Avantageusement, l'épaisseur de la section du renfort dorsal varie sensiblement régulièrement d'une extrémité à l'autre, et avantageusement est maximale vers la partie 5A et minimale vers le moyen 6, 6A.

Selon une autre version particulièrement avantageuse de l'invention, le cathéter 9 sera solidaire de l'embout 10, lui même solidaire de l'extrémité 4 de la bande souple 2, ledit embout étant surmoulé directement sur le cathéter 9.

Avantageusement, l'embout 10 sera réalisé en un matériau élastomère identique au premier matériau élastomère formant le renfort dorsal 12.

Les moyens de fermeture 5, 6 étant solidaires des extrémités 3, 4 de la bande souple 2 et s'étendant vers l'extérieur de ladite bande à partir du renfort dorsal 12, la chambre annulaire 7 se terminera avantageusement par deux sections transversales 15, 16 sensiblement planes, pour venir en appui l'une contre l'autre dans la position de fermeture de l'anneau (figure 2), de façon à former une chambre de compression annulaire 7 assurant une compression sur toute la périphérie de l'anneau, c'est-à-dire sur 360 degrés environ.

De manière particulièrement avantageuse, l'anneau de gastroplastie 1 conforme à l'invention sera réalisé avec une chambre annulaire 7, qui présente une section transversale de forme sensiblement elliptique. Cette particularité permet de conférer à la chambre une surface d'appui relativement large, en tout cas supérieure à celle des chambres annulaires classiques de section circulaire, en raison de la facilité de déformation élastique de la chambre due à la présence du second matériau élastomère particulièrement souple. Cette facilité de déformation, ainsi que la surface d'appui relativement large ou en tout cas augmentée, permettent de réduire la pression de contact entre l'estomac et l'anneau, du fait de l'augmentation relative de la surface de contact, ce qui réduit l'agression sur les tissus de l'estomac. Avantageusement, la section transversale elliptique sera sensiblement constante sur toute la longueur développée de la chambre annulaire 7.

De manière préférentielle, l'anneau de gastroplastie conforme à l'invention présentera également une mémoire de forme sensiblement circulaire, de manière à faciliter le positionnement de l'anneau par le chirurgien, puisque dans sa position de repos lâche ouverte (figure 1) l'anneau présente déjà une forme quasi ou sensiblement circulaire proche de sa position finale illustrée à la figure 2.

Enfin, tel qu'illustré notamment à la figure 3, le surmoulage du second matériau élastomère peut aboutir à la présence d'une légère épaisseur de matériau élastomère de rigidité inférieure (second matériau élastomère) sur et autour du renfort dorsal 12.

Le procédé de fabrication d'un anneau de gastroplastie conforme à l'invention est un procédé par injection d'au moins deux et dans le cas présent de deux matériaux élastomères de nature identique dans un moule pourvu d'au moins une empreinte comportant elle-même au moins un noyau.

Selon l'invention, le procédé de fabrication de l'anneau 1 est caractérisé en ce que :
- a) on assure l'injection d'un premier matériau élastomère de dureté Shore A déterminée d1 en vue de réaliser au moins le renfort dorsal de l'anneau,
- b) puis on assure par surmoulage sur au moins le renfort dorsal, l'injection d'un second matériau élastomère de même nature que le premier matériau, mais de dureté Shore A prédéterminée d2, avec d2 < d1, en vue de réaliser les parties restantes de l'anneau et obtenir ainsi un anneau monobloc surmoulé à dureté variable.

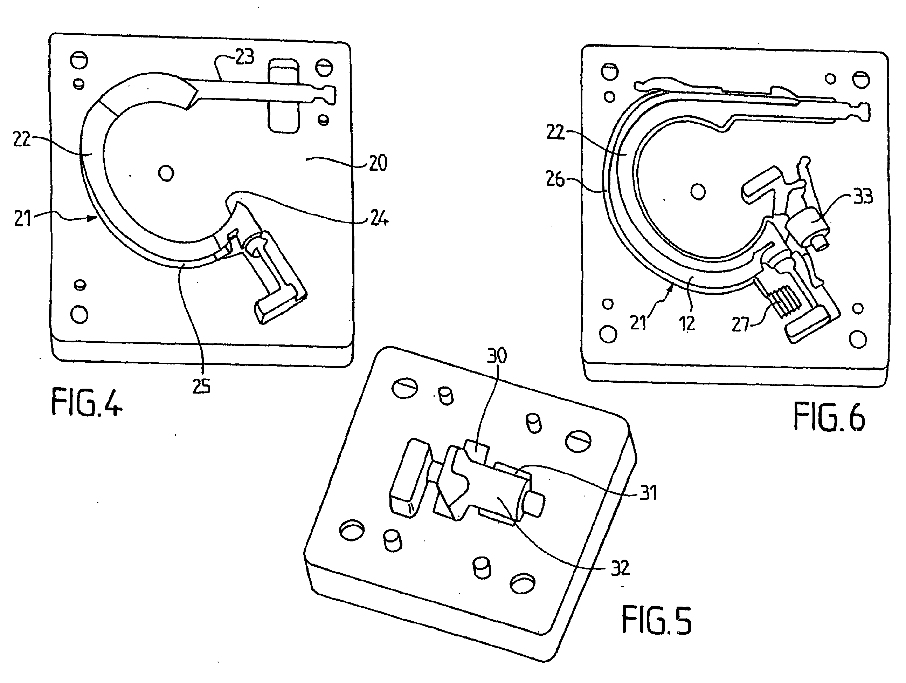

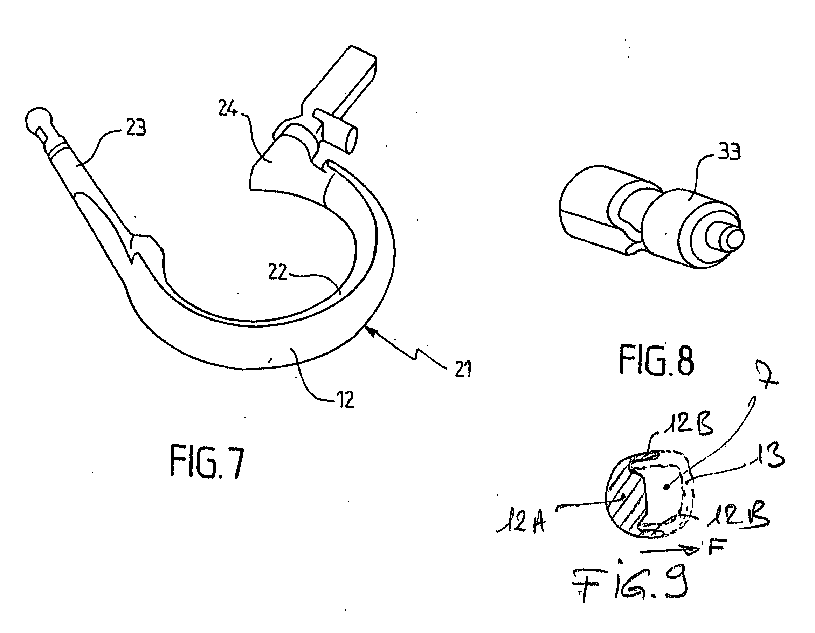

Tel qu'illustré à la figure 4, au cours de l'étape a), l'injection du premier matériau élastomère s'effectue dans une empreinte dorsale ménagée dans un moule 20, empreinte dorsale dans laquelle est disposé un noyau de renfort dorsal 21. Tel qu'illustré à la figure 4, le noyau de renfort dorsal 21 présente dans sa partie centrale 22 une forme sensiblement courbe correspondant à la pré-contrainte courbe souhaitée du renfort dorsal 12, ainsi que deux parties terminales 23, 24 correspondant respectivement à la partie mâle et femelle de l'anneau définitif. La partie centrale 22 présentera une section transversale sensiblement en U, de manière à former une gorge 25 destinée à recevoir la majorité du premier matériau élastomère injecté pour former l'arête principale du renfort dorsal 12. L'empreinte du moule est conjuguée à celle du noyau 21 de renfort dorsal, de telle manière que l'on obtient, à l'issue de l'étape a), un noyau de renfort dorsal 21 supportant le renfort dorsal 12, tel qu'illustré à la figure 7. Selon cette étape, le renfort dorsal 12 s'étend sur la majeure partie de la surface externe du noyau 21 et en particulier dans la gorge 25, et présente une section transversale en U, d'épaisseur variable et sensiblement régulière d'une extrémité à l'autre. Avantageusement, son épaisseur sera maximale à ou vers sa partie terminale 24, et diminuera régulièrement pour être minimale vers sa partie terminale 23. La partie centrale de compression reste cependant, avantageusement, d'épaisseur constante.

A l'issue de l'étape a), on retire le noyau de renfort 21 supportant le renfort dorsal 12 (figure 7) et on le place dans un autre moule (figure 6), dans lequel on a aménagé une empreinte d'anneau complète correspondant à la forme définitive de l'anneau 1.

Le procédé de fabrication selon l'invention peut ensuite se dérouler normalement, en assurant le surmoulage du reste de l'anneau sur le renfort dorsal 12 et sur le noyau 21 de renfort dorsal, par injection du second matériau élastomère pour obtenir l'anneau définitif monobloc et à rigidité variable (étape b).

Avantageusement, tel qu'illustré la figure 6, l'empreinte d'anneau 26 définitive pourra comporter, de préférence vers la partie dorsale de l'anneau correspondant à la partie 24 du noyau 21, une zone 27 d'empreinte susceptible de conduire à l'injection et à la formation d'un bouchon 28 (figure 3). Ce bouchon 28, à l'issue de l'opération de surmoulage du matériau élastomère de rigidité inférieure, est solidaire du corps de l'anneau définitif et sert à refermer de manière étanche, par exemple par collage, l'anneau lui-même qui comporte à cet endroit un orifice dû au passage des outils permettant l'extraction de l'anneau définitif et du noyau 21 de renfort dorsal à l'issue de l'étape a).

Selon une variante particulièrement avantageuse du procédé selon l'invention, il est envisageable au cours de l'étape a) d'assurer également la réalisation d'une partie des moyens de fermeture de l'anneau avec le premier matériau élastomère de dureté supérieure.

Cette étape spécifique qui permet de renforcer la solidité de l'anneau définitif, peut être réalisée dans un moule distinct (figure 5), dans lequel on a réalisé une empreinte du moyen de fermeture 30 comportant en particulier une zone spécifique 31 telle que, lorsqu'on dispose un noyau de bague 32 dans l'empreinte 30, la zone 31 permet d'obtenir une bague 33, telle qu'illustrée à la figure 8.

Ainsi, au cours de l'étape a), la réalisation d'une partie des moyens de fermeture de l'anneau s'effectue par injection du premier matériau de rigidité supérieure dans une empreinte 30, 31 du moyen de fermeture, en forme de bague, dans laquelle est disposé un noyau 32 de bague.

Ensuite, avant l'étape b), on retire le noyau de bague 32 supportant la bague 33 (figure 8) et on le place dans l'empreinte d'anneau 26 (figure 6) avec le noyau 21 de renfort dorsal ou destinée à recevoir ledit noyau de renfort dorsal. En effet, le noyau 21 de renfort dorsal peut être déjà en place dans l'empreinte d'anneau définitive ou au contraire être déposé après le dépôt du noyau 32 de bague.

Par la suite, l'injection du second matériau élastomère peut être effectuée de manière à surmouler par injection le second matériau de rigidité inférieure sur le renfort dorsal 12 et la bague 33 réalisée à base de matériau élastomère plus rigide.

Selon cette variante de réalisation préférentielle, la bague 33 forme la partie essentielle du moyen de fermeture femelle 5A.

Selon une autre variante de réalisation particulièrement intéressante, il est possible, au cours de l'étape b) de surmoulage et d'injection du second matériau élastomère, d'assurer également la réalisation de l'embout 10 de cathéter 9 qui est donc formé du second matériau élastomère de rigidité inférieure.

Cette opération de surmoulage (non représentée aux figures) est réalisée de manière classique dans un moule spécifique comportant une empreinte adaptée. Ainsi, on assure sur le cathéter 9, disposé dans l'empreinte de l'embout, l'injection du second matériau élastomère, de manière à obtenir un embout 10 surmoulé sur le cathéter 9.

Puis, on retire le cathéter 9 supportant l'embout 10 et on assemble, par exemple par collage, ledit embout avec l'anneau définitif obtenu à l'issue de l'étape b), tel qu'illustré par exemple à la figure 6.

Le procédé tel que décrit précédemment permet d'obtenir une très bonne adhésion respective des matériaux élastomères surmoulés et ce, dans un temps relativement bref, l'ensemble des opérations étant facilement automatisable et faisant intervenir un minimum d'opérations manuelles.

On obtient également un anneau monobloc présentant une grande régularité dans tous ces éléments le composant.

L'invention concerne également une méthode de traitement thérapeutique de l'obésité morbide incluant les étapes d'installation, de contrôle et de réglage notamment du diamètre, d'un anneau gastrique conforme à l'invention.

### POSSIBILITE D'APPLICATION INDUSTRIELLE

L'invention trouve son application industrielle dans la conception et la fabrication d'anneaux gastriques destinés au traitement de l'obésité.

## Revendications

1. - Anneau de gastroplastie (1) formé par une bande souple (2) destinée à être fermée vers ses deux extrémités (3, 4) par des moyens de fermeture (5, 6) autour de l'estomac d'un patient pour réduire le diamètre de l'ouverture du stoma, ladite bande comportant une chambre de compression annulaire (7) à volume réglable reliée par un cathéter (9) à un dispositif de réglage du diamètre de ladite chambre par injection ou retrait de fluide, ladite chambre étant délimitée par des parois comprenant un renfort dorsal (12) prolongé par des parois latérales (13), **caractérisé en ce que** :
- le renfort dorsal (12) est réalisé à partir d'un premier matériau élastomère de dureté Shore A, prédéterminée d₁,
- les parois latérales (13) sont réalisées à partir d'un second matériau élastomère de même nature que le premier matériau mais de dureté Shore A prédéterminée d₂, telle que d₂ < d₁, de manière à obtenir une chambre annulaire (7) monobloc de dureté Shore A variable dans son épaisseur.

2. - Anneau selon la revendication 1 **caractérisé en ce que** la chambre annulaire (7) est obtenue par une opération de surmoulage des deux matériaux élastomères, les parois latérales (13) étant surmoulées sur le renfort dorsal (12).

3. - Anneau selon la revendication 1 ou 2 **caractérisé en ce que** la valeur de d₁ est comprise entre 65 et 85 Shore A, la valeur de d₂ étant comprise entre 25 et 45 Shore A.

4. - Anneau selon la revendication 3 **caractérisé en ce que** la valeur de d₁ est de l'ordre de 80 Shore A, la valeur de d₂ est de l'ordre de 30 Shore A.

5. - Anneau selon l'une des revendications 1 à 4 **caractérisé en ce qu'**une partie des moyens de fermeture (5, 6) est réalisée à partir du premier matériau élastomère.

6. - Anneau selon la revendication 5 **caractérisé en ce que** les moyens de fermeture (5, 6) comprennent un moyen femelle (5A) solidaire d'une extrémité (3) de la bande souple (12) destiné à coopérer avec un moyen mâle (6) solidaire d'une autre extrémité de la bande souple, le moyen femelle étant formé par une bague (5A) réalisée à partir du premier matériau élastomère.

7. - Anneau selon l'une des revendications 1 à 6 **caractérisé en ce que** le cathéter (9) comporte un embout (10) solidaire d'une extrémité de la bande souple, ledit embout étant surmoulé sur le cathéter (9).

8. - Anneau selon la revendication 7 **caractérisé en ce que** l'embout (10) est réalisé à partir du premier matériau.

9. - Anneau selon l'une des revendications 1 à 8 **caractérisé en ce que** les moyens de fermeture (5. 6) sont solidaires des extrémités (3, 4) de la bande souple (2) et s'étendent vers l'extérieur de la bande à partir du renfort dorsal (12), la chambre annulaire (7) se terminant par deux sections transversales (15, 16) sensiblement planes pour venir en appui l'une contre l'autre dans la position de fermeture de l'anneau, de façon à former une chambre de compression annulaire (7) assurant une compression sur toute la périphérie de l'anneau.

10. -Anneau selon l'une des revendications 1 à 9 **caractérisé en ce que** la chambre annulaire (7) présente une section transversale de forme sensiblement elliptique.

11. -Anneau selon l'une des revendications 1 à 10 **caractérisé en ce qu'**il présente une mémoire de forme sensiblement circulaire.

12. - Anneau selon l'une des revendications 1 à 11 **caractérisé en ce que** le renfort dorsal (12) présente une section transversale sensiblement en U.

13. - Anneau selon la revendication 12 **caractérisé en ce que** l'âme (12A) du renfort dorsal (12) est d'épaisseur supérieure à celle des branches (12B).

14. -Procédé de fabrication d'un anneau de gastroplastie par injection d'un matériau élastomère dans un moule pourvu d'au moins une empreinte avec au moins un noyau, **caractérisé en ce que** :
- a) on assure l'injection d'un premier matériau élastomère de dureté Shore A prédéterminée d₁ en vue de réaliser au moins le renfort dorsal de l'anneau ;
- b) puis on assure, par surmoulage sur au moins le renfort dorsal, l'injection d'un second matériau élastomère de même nature que le premier matériau mais de dureté Shore A prédéterminée d₂, avec d₂ < d₁, en vue de réaliser les parties restantes de l'anneau et obtenir un anneau monobloc surmoulé à dureté variable.

15. -Procédé selon la revendication 14 **caractérisé en ce qu'**au cours de l'étape a), on assure également la réalisation d'une partie des moyens de fermeture de l'anneau avec le premier matériau élastomère.

16. -Procédé selon la revendication 14 ou 15 **caractérisé en ce qu'**on assure également la réalisation d'un embout de cathéter avec le second matériau élastomère.

17. -Procédé selon la revendication 14 **caractérisé en ce que** :
- au cours de l'étape a), l'injection du premier matériau élastomère s'effectue dans une empreinte dorsale dans laquelle est disposé un noyau de renfort dorsal ;
- puis on retire le noyau de renfort dorsal supportant le renfort dorsal et on le place dans une empreinte d'anneau ;
- puis on assure le déroulement de l'étape b) pour obtenir l'anneau définitif.

18. -Procédé selon les revendications 15 et 17 **caractérisé en ce que** :
- au cours de l'étape a), la réalisation d'une partie des moyens de fixation de l'anneau s'effectue par injection du premier matériau dans une empreinte du moyen de fixation en forme de bague dans laquelle est disposé un noyau de bague ;
- puis, avant l'étape b), on retire le noyau de bague supportant la bague et on le place dans l'empreinte d'anneau avec le noyau de renfort dorsal ou destinée à recevoir ledit noyau de renfort dorsal.

19. -Procédé selon les revendications 16 et 18 **caractérisé en ce que** :
- au cours de l'étape b), on assure la réalisation de l'embout par injection du second matériau dans une empreinte de cathéter comportant un cathéter ;
- puis on retire le cathéter supportant l'embout et on assemble, par exemple par collage, ledit embout avec l'anneau définitif obtenu à l'issue de l'étape b).

## Claims

1. Gastroplasty ring (1) formed by a flexible band (2) intended to be closed towards its two ends (3, 4) by means (5, 6) of closure about the stomach of a patient in order to reduce the diameter of the stoma opening, the said band comprising an annular compression chamber (7) with an adjustable volume connected by a catheter (9) to a device for adjusting the diameter of the said chamber by injecting or withdrawing fluid, the said chamber being delimited by walls comprising a dorsal reinforcement (12) extended by lateral walls (13), **characterised in that**:
- the dorsal reinforcement (12) is produced from a first elastomer material with a predetermined Shore A hardness d₁,
- the lateral walls (13) are produced from a second elastomer material of the same nature as the first material but with a predetermined Shore A hardness d₂, such that d₂ < d₁, so as to obtain a single-piece annular chamber (7) with a variable Shore A hardness in its thickness.

2. Ring according to claim 1, **characterised in that** the annular chamber (7) is obtained by an operation of insert moulding of the two elastomer materials, the lateral walls (13) being moulded onto the dorsal reinforcement (12).

3. Ring according to claim 1 or 2, **characterised in that** the value of d₁ is between 65 and 85 Shore A, the value of d₂ being between 25 and 45 Shore A.

4. Ring according to claim 3, **characterised in that** the value of d₁ is around 80 Shore A, and the value of d₂ is around 30 Shore A.

5. Ring according to one of claims 1 to 4, **characterised in that** part of the closure means (5, 6) is produced from the first elastomer material.

6. Ring according to claim 5, **characterised in that** the closure means (5, 6) comprise a female means (5A) fixed to one end (3) of the flexible band (12) intended to cooperate with a male means (6) fixed to the other end of the flexible band, the female means being formed by a hoop (5A) produced from the first elastomer material.

7. Ring according to one of claims 1 to 6, **characterised in that** the catheter (9) comprises a connecting piece (10) fixed to one end of the flexible band, the said connecting piece being moulded onto the catheter (9).

8. Ring according to claim 7, **characterised in that** the connecting piece (10) is produced from the first material.

9. Ring according to one of claims 1 to 8, **characterised in that** the closure means (5, 6) are fixed to the ends (3, 4) of the flexible band (2) and extend towards the outside of the band from the dorsal reinforcement (12), the annular chamber (7) terminating in two substantially planar transverse sections (15, 16) in order to come into abutment against each other in the closure position of the ring, so as to form an annular compression chamber (7) providing compression over the entire periphery of the ring.

10. Ring according to one of claims 1 to 9, **characterised in that** the annular chamber (7) has a transverse section that is substantially elliptical in shape.

11. Ring according to one of claims 1 to 10, **characterised in that** it has a substantially circular shape memory.

12. Ring according to one of claims 1 to 11, **characterised in that** the dorsal reinforcement (12) has a transverse section substantially in a U shape.

13. Ring according to claim 12, **characterised in that** the web (12A) of the dorsal reinforcement (12) has a thickness greater than that of the branches (12B).

14. Method of manufacturing a gastroplasty ring by injecting an elastomer material into a mould provided with at least one cavity with at least one core, **characterised in that**:
- a) a first elastomer material with a predetermined Shore A hardness d₁ is injected with a view to producing at least the dorsal reinforcement of the ring;
- b) then, by insert moulding on at least the dorsal reinforcement, a second elastomer material of the same nature as the first material but with a predetermined Shore A hardness d₂ is injected, with d₂ < d₁, with a view to producing the remaining parts of the ring and obtaining a moulded-on single-piece ring of variable hardness.

15. Method according to claim 14, **characterised in that**, during step a), part of the closure means of the ring are also produced with the first elastomer material.

16. Method according to claim 14 or 15, **characterised in that** a catheter connecting piece is also produced with the second elastomer material.

17. Method according to claim 14, **characterised in that**:
- during step a), the first elastomer material is injected in a dorsal cavity in which a dorsal reinforcement core is disposed;
- then the dorsal reinforcement core supporting the dorsal reinforcement is removed and is placed in a ring cavity;
- then step b) is performed in order to obtain the definitive ring.

18. Method according to claims 15 and 17, **characterised in that**:
- during step a), part of the fixing means of the ring is produced by injecting the first material into a cavity of the fixing means in the form of a hoop in which a hoop core is disposed;
- then, before step b), the hoop core supporting the hoop is removed and is placed in the ring cavity with the dorsal reinforcement core or intended to receive the said dorsal reinforcement core.

19. Method according to claims 16 and 18, **characterised in that**:
- during step b), the connecting piece is produced by injecting the second material in a catheter cavity comprising a catheter;
- then the catheter supporting the connecting piece is removed and the said connecting piece is assembled, for example by adhesive bonding, with the definitive ring obtained at the end of step b).

## Patentansprüche

1. Gastroplastie-Ring (1) aus einem flexiblen Band (2) gebildet, das so ausgeführt ist, dass es an seinen beiden Enden (3,4) durch Verschlussmittel (5,6) um den Magen eines Patienten schließt, um den Durchmesser der Stomaöffnung zu reduzieren, wobei das Band eine ringförmige Kompressionskammer (7) mit verstellbarem Volumen umfasst, die über einen Katheter (9) mit einer Vorrichtung zur Regelung des Durchmessers dieser Kammer durch Injektion oder Entzug von Flüssigkeit verbunden ist, wobei diese Kammer durch Wände abgegrenzt ist, die eine Rückenverstärkung (12) umfassen, die durch Seitenwände (13) verlängert ist, **dadurch gekennzeichnet, dass**
- die Rückenverstärkung (12) aus einem ersten Elastomermaterial mit einem vorbestimmten Shore A Härtegrad d₁ ausgebildet ist,
- die Seitenwände (13) aus einem zweiten Elastomennaterial derselben Art wie das erste Material, aber mit einem vorbestimmten Shore A Härtegrad d₂, wobei d₂ < d₁ ist, ausgebildet sind, um eine einstückige ringförmige Kammer (7) mit in seiner Dicke variablem Shore A Härtegrad zu erhalten.

2. Ring nach Anspruch 1, **dadurch gekennzeichnet, dass** die ringförmige Kammer (7) durch einen Vorgang des Übergießens der beiden Elastomermaterialien erzeugt wird, wobei die Seitenwände (13) auf der Rückenverstärkung (12) übergossen sind.

3. Ring nach Anspruch 1 oder 2, **dadurch gekennzeichnet, dass** der Wert d₁ zwischen 65 und 85 Shore A liegt und der Wert d₂ zwischen 25 und 45 Shore A liegt.

4. Ring nach Anspruch 3, **dadurch gekennzeichnet, dass** der Wert d₁ 80 Shore A entspricht und der Wert d₂ 30 Shore A entspricht.

5. Ring nach einem der Ansprüche 1 bis 4, **dadurch gekennzeichnet, dass** ein Teil der Verschlussmittel (5, 6) aus dem ersten Elastomermaterial ausgebildet ist.

6. Ring nach Anspruch 5, **dadurch gekennzeichnet, dass** die Verschlussmittel (5, 6) eine Steckeraufnahme (5A) umfassen, die mit einem Ende (3) des flexiblen Bandes (12) verbunden ist, die dazu bestimmt ist, mit einem Stecker (6) zusammenzuwirken, der mit einem anderen Ende des flexiblen Bandes verbunden ist, wobei die Steckeraufnahme aus einem Ring (5A) gebildet ist, welcher aus dem ersten Elastomermaterial ausgebildet ist.

7. Ring nach einem der Ansprüche 1 bis 6, **dadurch gekennzeichnet, dass** der Katheter (9) ein Ansatzstück (10) umfasst, das mit einem Ende des flexiblen Bandes verbunden ist, wobei das Ansatzstück auf dem Katheter (9) übergossen ist.

8. Ring nach Anspruch 7, **dadurch gekennzeichnet, dass** das Ansatzstück (10) aus dem ersten Material besteht.

9. Ring nach einem der Ansprüche 1 bis 8, **dadurch gekennzeichnet, dass** die Verschlussmittel (5,6) mit den Enden (3,4) des flexiblen Bandes (2) verbunden sind und sich außerhalb des Bandes von der Rückenverstärkung (12) aus erstrecken, wobei die ringförmige Kammer (7) durch zwei im Wesentlichen ebene Querschnitte (15, 16) abgeschlossen ist, um aufeinander in der Schließposition des Ringes aufzuliegen, damit eine ringförmige Kompressionskammer (7) gebildet wird, die eine Kompression auf der gesamten Peripherie des Ringes sicherstellt.

10. Ring nach einem der Ansprüche 1 bis 9, **dadurch gekennzeichnet, dass** die ringförmige Kammer (7) einen Querschnitt mit einer im Wesentlichen elliptischen Form aufweist.

11. Ring nach einem der Ansprüche 1 bis 10, **dadurch gekennzeichnet, dass** er ein im Wesentlichen kreisförmiges Formgedächtnis aufweist.

12. Ring nach einem der Ansprüche 1 bis 11, **dadurch gekennzeichnet, dass** die Rückenverstärkung (12) einen im Wesentlichen U-formigen Querschnitt aufweist.

13. Ring nach Anspruch 12, **dadurch gekennzeichnet, dass** die Seele (12A) der Rückenverstärkung (12) eine höhere Dicke als die der Ausläufer (12B) hat.

14. Verfahren zur Herstellung eines Gastroplastie-Ringes durch Einspritzen eines Elastomermaterials in eine Form, ausgestattet mit mindestens einer Vertiefung mit mindestens einem Kern, **dadurch gekennzeichnet, dass**
a) das Einspritzen eines ersten Elastomermaterials mit einem vorbestimmten Shore A Härtegrad d₁ durchgeführt wird, um mindestens die Rückenverstärkung des Ringes zu realisieren;
b) dann durch Übergießen auf mindestens der Rückverstärkung das Einspritzen eines zweiten Elastomermaterials derselben Art wie das erste Material, aber mit einem vorbestimmten Shore A Härtegrad d₂, wobei d₂ <d₁ ist, ausgeführt wird, um die restlichen Teile des Ringes zu realisieren und einen einstückigen übergossenen Ring mit variablem Härtegrad zu erhalten.

15. Verfahren nach Anspruch 14, **dadurch gekennzeichnet, dass** im Laufe des Schritts a) auch die Herstellung eines Teils der Verschlussmittel des Ringes mit dem ersten Elastomermaterial ausgeführt wird.

16. Verfahren nach Anspruch 14 oder 15, **dadurch gekennzeichnet, dass** auch die Herstellung eines Katheteransatzstücks mit dem zweiten Elastomermaterial ausgeführt wird.

17. Verfahren nach Anspruch 14, **dadurch gekennzeichnet, dass**
- im Laufe der Schritts a) das Einspritzen des ersten Elastomermaterials in eine Rückenvertiefung ausgeführt wird, in welcher ein Kern für die Rückenverstärkung vorgesehen ist;
- dann der Kern für die Rückenverstärkung, der die Rückenverstärkung stützt, entfernt und in eine Ringvertiefung gelegt wird;
- dann der Ablauf des Schritts b) durchgeführt wird, um den endgültigen Ring zu erhalten.

18. Verfahren nach den Ansprüchen 15 und 17, **dadurch gekennzeichnet, dass**
- im Laufe des Schritts a) die Herstellung eines Teils der Befestigungsmittel des Ringes durch Einspritzen des ersten Materials in eine ringförmige Vertiefung des Befestigungsmittels durchgeführt wird, in welcher ein Ringkern vorgesehen ist;
- dann vor dem Schritt b) der Ringkern, der den Ring stützt, entfernt und in die Ringvertiefung mit dem Kern für die Rückenverstärkung gelegt wird, oder die dafür vorgesehen ist, den besagten Kern für die Rückenverstärkung aufzunehmen.

19. Verfahren nach den Ansprüchen 16 und 18, **dadurch gekennzeichnet, dass**
- im Laufe des Schritts b) die Herstellung des Ansatzstückes durch Einspritzen des zweiten Materials in eine Kathetervertiefung, die einen Katheter enthält, ausgeführt wird;
- dann der das Ansatzstück stützende Katheter entfernt und z. B. durch Kleben das besagte Ansatzstück mit dem endgültigen Ring verbunden wird, der am Ende des Schritts b) erzielt wurde.
